# EUROPEAN PATENT APPLICATION

(11) **EP 0 803 202 A2**
(43) Date of publication of application: **29.10.1997**
(21) Application number: 97830189.3
(22) Date of filing: 24.04.1997
(51) Int. Cl.: A23L 1/30, A23L 1/304, A23L 1/308

(54) **Dietary composition containing chitosan, Garcinia cambogia hydroxycitrate and organic chromium**

(30) Priority: 26.04.1996 IT RM960279
(71) Applicant: SIRC S.p.A. NATURAL & DIETETIC FOODS, I-20090 Caleppio Di Settala (MI) (IT)
(72) Inventor: Littera, Renato, 10143 Torino (IT)
(74) Representative: Sarpi, Maurizio

(57) **Abstract**

The use of preparations based on the combination of chitosan with organic chromium and Garcinia cambogia hydroxycitrate as dietary products for the treatment of obesity having hypocholesteremic and sugar absorption reducing activity is disclosed.
The proposed combination of chitosan with organic chromium and Garcinia cambogia hydroxycitrate is formulated on the base of the effects that the above three components have on the glucid metabolism. Such effects tends particularly to decrease the values of cholesterolemia and triglycerides in case they are too high.
The integrator of the invention can be administered by mouth in the usual dose unit both as capsules and tablets and is efficacious as diet integrator in the weight reducing programs aiming at calorie restrictions in obese subjects, in the treatment of hypertension, and as hypocholesteremic product.

## Description

The present invention relates to a chitosan product against cholesterol.
More particularly, the invention relates to the use of preparations based on the combination of chitosan with organic chromium and Garcinia cambogia hydroxycitrate as dietary products having hypocholesteremic and sugar absorption reducing activity for the treatment of the obesity.
The proposed combination of chitosan with organic chromium and Garcinia cambogia hydroxycitrate is formulated on the base of the effects that the above three components have on the glucid metabolism. Such effects tends particularly to decrease the values of cholesterolemia and triglycerides in case they are too high.
The integrator of the invention can be administered by mouth in the usual dose unit both as capsules and tablets and is efficacious as diet integrator in the weight reducing programs aiming at calorie restrictions in obese subjects, in the treatment of hypertension, and as hypocholesteremic product.

### CHITOSAN

Chitosan, widely described in the literature as early as the beginning of last century, is a water-soluble, non-acetylated or partially deacetylated derivative of chitin (linear homopolymer of β-(1,4)-N-acetyl-glucosamine) which is one of the main natural constituents of the exoskeleton, the hard shell of the crustaceans.
Chitosan has important chemical, biochemical properties which have attracted the interest of a number of researchers for some time. Recent studies have particularly pointed out the hypocholesteremic effect of chitosan which is capable of interfering directly with the intestinal absorption of lipids such as cholestyramine without toxic effects (see Jennings C.D. et al. "A comparison of the lipid-lowering and intestinal morphological effects of cholestyramine, chitosan and oat gum in rats" in Proc.Soc.Biol.Med. 189 (1) :13,1988).
Such antihypercholesteremic, antihyperlipemic effects are due to the inhibition of fat digestion (see Deuchi K. et al. "Effect of the viscosity or deacetylation degree of chitosan on fecal fat excreted from rats fed on a high-fat diet" in Biosci. Biotechnol.Bioche (Japan) 59 (5) :781,1995). It was found that the polycationic chitosan fibre can bind through its positive charged aminic groups the carboxylic groups of fatty acids having negative charge so as to form ionic bonds; the neutral fats such as triglycerides, cholesterol or alimentary sterols are bound by hydrophobic bonds. After ingestion, chitosan is solubilized in the acid environment of the stomach and then changed to a gelled form capable of entrapping fats, thus decreasing the intestinal fat absorption and causing a large amount of fat excretion in the faeces.
In addition to a reduction of the total cholesterol in obese patients treated with chitosan it was also found a significant increase of the HDL cholesterol (bound to high-density lipoprotein) which as known is inversely correlated to the cardiovascular diseases.

### GARCINIA CAMBOGIA DESR.

Garcinia cambogia (family of Guttiferae) is a spontaneous plant which grows in the tropical climate zones of South India, Indochina, Cambodia and Philippines and produces large yellowish fruits (berries) having the form of pumpkins. The rind of the fruit, known as "Malabar Tamarind" or "Goraka" and used for centuries by South Asia people as flavour enhancer and digestion assistant, is full of an acid identified only by the half of years '60 as hydroxycitric acid or hydroxycitrate. The hydroxycitrate acts on the liver and on other regions (adipose tissue, small intestine, etc.) and reduces the production of cholesterol and triglycerides (up to 27%) from the sugars. The hydroxycitrate operates as follows: the final destruction of ingested sugars, proteins and fats takes place in the mitochondria through the cycle of citric acid with production of energy through the respiratory chain directly connected thereto. The molecules of glucose which are not immediately used for producing energy are stored in the liver and the muscles under the form of glycogen. When the glycogen supplies are saturated, the exceeding glucose molecules are further divided and converted into fat and cholesterol by an enzyme called ATP citrate lyase. The hydroxycitrate acts temporarily as inhibitor of this enzyme. Studies conducted on animals showed that the hydroxycitrate reduces the fatty acid synthesis by about 40-70% during 8-12 hours after meals.
At the same time, since the hydroxycitrate inhibits the enzyme ATP citrate lyase, the glucose molecules which do not transform into fat or cholesterol produce further glycogen. As the glycogen levels increase, the body sends a satiety signal to brain which in turn suppresses the appetite sensation so as to cause a minor food assumption.

### ORGANIC CHROMIUM

The scientific literature showing the importance of chromium in the human nourishing is very wide (J. Lederer: Le Chrome, Cah.Nutr.Diet., XXIV 2. 1989, 112-118), particularly regarding the check of glucid and lipid metabolism as well as the insufficient supply of chromium with the diet which needs a dietary chromium supplementation.
Anderson et al. (Serum chromium of human subjects: effects of chromium supplementation and glucose. The American Journal of Clinical Nutrition. 41: March 1985, pages 571-577) in a double-blind study conducted with 3 groups of subjects with different tolerances to glucose relate a clear improvement of such tolerance as a result of an exhibition of 200 micrograms/day of chromium under the form of chromium chloride. The authors further relate an increase in the glycaemia level in hypoglycaemic subjects treated with the same chromium supplement.
As far as the effect of chromium on the lipid metabolism is concerned, there are different experimental studies on animals and humans which indicate a bond between marginal lacks of chromium and alterations of lipid metabolism.
Thus, for example, rats fed with diets full of carbohydrates and cholesterol and lacking in chromium showed a hyperlipoproteinemia, which was considerably defeated by supplementing them with a diet based on chromium.
Particularly, total cholesterolemia, triglycerides and LDL cholesterol levels were reduced, while HDL choelesterol levels were increased. In hypercholesteremic rabbit Abrahem et al. observed a minor occurrence and consistency of atherosclerotic plaques as a result of an exhibition of chromium.
In human trials it was found that the oral exhibition of 20 micrograms of chromium to 12 volunteers during three months caused a reduction of triglycerides and an increase in the plasmatic level of HDL cholesterol with respect to the controls.
Also Mossop's study on diabetic subjects fed with a dietary supplement of chromium showed, in addition to a hypoglycaemic effect, a reduction of the total cholesterolemia and an increase by 25% in HDL cholesterol.

There are also studies reporting a beneficial effect of the supplementation with yeast containing the biologically active form of chromium on the lipid concentration.
A significant correlation between occurrence of the coronary disease and reduced plasmatic chromium levels has been observed in subjects suffering from cardiovascular diseases.
The concentration of chromium in aorta, myocardium, kidney and hair of subjects dead of myocardial infarction was much lower than the concentration of chromium in patients dead by other reasons.
On the base of such experimental, clinical data it is possible to infer that the chromium exerts through the regulation of the lipid metabolism a protection against the occurrence and the development of atherosclerotic processes with reduction of the mortality risk due to cardiovascular diseases.
The use of organic chromium in combination with antioxidant vitamins such as vitamin E and vitamin C in dietary preparations for preventing cardiovascular diseases is described in the Italian Patent No. 1254311 of 3.20.1992 in the name of the same Applicant to which reference is made.
The conducted experiments showed that the best results are attained with daily exhibition of 100-200 micrograms of Cr. In the preferred dose unit of 350 mg, where the chromium and vitamin content is sufficient for a daily average dose of only one capsule per day, the three active components are present in the following weight proportions:

| | |
|---|---|
| organic chromium | 40-65%, |
| vitamin C | 24-40%, |
| tocopherol acetate | 8-18%, |

where the organic chromium is under the form of chromium yeast in the amount of 75 mg corresponding to 150 micrograms of chromium.
In recent studies of the Applicant on the base of scientific experimental data, a new combination among purified chitosan, organic chromium and Garcinia cambogia hydroxycitrate was found adapted for preparing a dietary product having a surprising hypocholesteremic and sugar absorption reducing activity. In such combination, the three active components are present in the following weight proportions:

| | |
|---|---|
| chitosan | 65-75% |
| Garcinia cambogia hydroxycitrate | 15-20% |
| organic chromium | 5-6%. |

Several controlled clinic trials showed the validity of a dose unit under the form of a capsule in which the content of chitosan and other substances is sufficient for a daily average exhibition of two capsules.
Alternately, a dose unit under the form of tablet sufficient for a daily average exhibition of one tablet is provided.

The present invention is illustrated by the following not limiting formulation.

| A) CAPSULE FOR ORAL EXHIBITION | |
|---|---|
| Purified chitosan | 240 mg |
| Garcinia cambogia hydroxycitrate | 55 mg |
| Organic chromium | 19 mg |

| B) TABLET FOR ORAL EXHIBITION | |
|---|---|
| Purified chitosan | 480 mg |
| Garcinia cambogia hydroxycitrate | 110 mg |
| Organic chromium | 38 mg |

The effect of the dietary integrator dose under the form of capsules of formulation A on the antihyperlipoproteinemic activity was the object of a study conducted on 150 obese patients who used the same during a dietary program.
The study was of the random, double-blind placebo-controlled type, in which the patients were treated with a hypocaloric diet plus 2 capsules/day of placebo (50 patients) or with a hypocaloric diet plus 1 capsule/day of placebo plus 1 capsule/day of dietary integrator (50 patients) or with a hypocaloric diet plus 2 capsule/day of dietary integrator (50 patients) during 4 weeks.
Table 1 shows the variations in the significant parameters of the three groups of patients and the statistical analysis.
The study pointed out in all of the experimental groups a statistically significant reduction, related to the dose of administered dietary integrator, of the overweight, total cholesterol, LDL cholesterol and triglycerides, and an increase in HDL cholesterol.

**Table 1**

| Characteristics of the treated patients | | | | |
|---|---|---|---|---|
| | Placebo | Integrator (1 capsule/day) | Integrator (2 capsules/day) | Statistical comparison |
| Patients (No.) | 50 | 50 | 50 | |
| Male (No.) | 22 | 23 | 26 | N.S. * |
| Female (No.) | 28 | 27 | 24 | N.S. * |
| Age (years) | 42.3±11.6 | 40.8±10.2 | 43.2±13.1 | N.S. ** |
| Weight (kg) | 86.2±8.3 | 85.9±7.6 | 87.1±8.7 | N.S. ** |
| Height (cm) | 171.4±6.2 | 174.5±7.1 | 172.8±7.4 | N.S. ** |
| Overweight | 21.4±4.2 | 19.9±3.8 | 20.6±4.9 | N.S. ** |
| Total Cholesterol (mgldl) | 285.6±37.6 | 296.2±33.8 | 291.5±40.1 | N.S. ** |
| LDL Cholesterol (mgldl) | 194.6±35.9 | 190.3±31.4 | 198.1±39.7 | N.S. ** |
| HDL Cholesterol (mgldl) | 27.2±6.5 | 25.7±7.3 | 26.4±6.2 | N.S. ** |
| Triglycerides (mgldl) | 235.8±37.6 | 244.2±40.3 | 241.5±42.1 | N.S. ** |
| Means ± d.s. | | | | |

| | | | | |
|---|---|---|---|---|
| * Not significant. CHI-square test. | | | | |
| ** Not significant. Student's t-test. | | | | |

In the group treated with placebo the overweight was reduced by 4.3%, the total cholesterol was reduced by 10.7%, LDL cholesterol was reduced by 15.2%, triglycerides were reduced by 13.2%, and HDL cholesterol was increased by 6.3%.
In the group treated with 1 capsule/day of the dietary integrator the overweight was reduced by 7.9%, the total cholesterol was reduced by 19.6%, LDL cholesterol was reduced by 22.9%, triglycerides were reduced by 18.3%, and HDL cholesterol was increased by 9.0%.
In the group treated with 2 capsules/day of dietary integrator the overweight was reduced by 12.5%, the total cholesterol was reduced by 28.7%, LDL cholesterol was reduced by 35.1%, triglycerides were reduced by 26.6%, and HDL cholesterol was increased by 14.1%.
Side effects were observed in 6.4% of patients treated with placebo (sickness and/or constipation), in 6.1% of patients treated with 1 capsule/day of dietary integrator (sickness), and in 2.1% of patients treated with 2 capsules/day of dietary integrator (cephalea) without any statistically significant difference among the three groups.
No pathological or clinically significant changes of the examined haematologic, haematochemical parameters were observed.
It is evident from the results that the exhibition of a dietary integrator including 240 mg of chitosan, 80 mg of Garcinia cambogia hydroxycitrate, and 19 mg of organic chromium during a hypocaloric program of weight reduction in obese patients can attain a significant reduction of overweight and hyperlipoproteinemia as well as a significant beneficial increase in the HDL cholesterol. The variations in the groups treated with the dietary integrator are greater than those provided only by a hypocaloric diet and depend on the administered dose of dietary integrator.
The obtained result may be ascribed to a reduction of fat digestion and to the intestinal absorption of the same, the higher level of fats excreted in the faeces being due to chitosan and to the reduction of the food consumption and fat acid synthesis induced by the hydroxycitric acid present in Garcinia cambogia extract. The reduction of the total cholesterol, LDL cholesterol and triglycerides due to the synergistic effect of the organic chromium is similar to that of cholestyramine but the tolerability of the product is undoubtedly better. Therefore, the use of a dietary product based on the combination of chitosan, Garcinia cambogia extract and chromium can be advantageously indicated in all of the weight reduction programs, thus allowing at the same time a parallel check of the hyperlipidemia.

## Claims

1. Use of chitosan in combination with Garcinia cambogia extract and chromium in dietary preparations for reducing the total cholesterol, LDL cholesterol, triglycerides and/or the body weight, and for increasing HDL cholesterol.

2. Use of chitosan in combination with Garcinia cambogia hydroxycitrate and organic chromium in dietary preparations having antihyperlipoproteinemic, anticholesteremic activity.

3. A dietary preparation for the treatment of hyperlipoproteinemia in obese subjects including chitosan in combination with Garcinia cambogia hydroxycitrate and organic chromium.

4. The dietary preparation of claim 3, characterized in that the active components are present in the dose unit in the following weight proportions:
| | |
|---|---|
| chitosan | 65-75% |
| Garcinia cambogia hydroxycitrate | 20-25% |
| organic chromium | 5-6%. |

5. The dietary preparation of claims 3, characterized in that the dose unit is a capsule containing:
| | |
|---|---|
| chitosan | 240 mg |
| Garcinia cambogia extract | 55 mg |
| organic chromium | 19 mg. |

6. The dietary preparation of claims 3 to 5, characterized in that the dose unit is a capsule containing:
| | |
|---|---|
| chitosan | 480 mg |
| Garcinia cambogia extract | 110 mg |
| organic chromium | 38 mg. |
